# EUROPEAN PATENT APPLICATION

(11) **EP 3 808 170 A1**
(43) Date of publication of application: **21.04.2021**
(21) Application number: 19203911.3
(22) Date of filing: 17.10.2019
(51) Int. Cl.: A01H 1/04, A01H 5/10, A01H 6/14

(54) **LACTUCA SATIVA RESISTANCE TO BREMIA LACTUCAE**

(71) Applicant: Bejo Zaden B.V., 1749 CZ Warmenhuizen (NL)
(72) Inventor: KAANDORP, Stefanus Johannes, 1906CT Limmen (NL); CHRISTIANEN, Claudia Jacoba Wilhelmina, 4845 PD Wagenberg (NL); SCHEURWATER, Teunis, 2957 NH Nieuw-Lekkerland (NL); VEENSTRA, Roelof Marinus, 1766 HC Wieringerwaard (NL); SCHRIJVER, Albertus Johannes Maria, 1749 KH Warmenhuizen (NL)
(74) Representative: Brantsandpatents bvba

(57) **Abstract**

The current invention relates to a nucleic acid fragment comprising a resistance allele from *L. serriola* which confers a broad spectrum resistance to *Bremia lactucae,* wherein, said resistance allele co-segregates with one or more marker sequences chosen from Seq ID No. 1, Seq ID No. 2, Seq ID No. 3, Seq ID No. 4, Seq ID No. 5, Seq ID No. 6, Seq ID No. 7, and/or any *L. serriola* genome specific marker in between Seq ID No. 1 and Seq ID No. 7.

SEQUENCE LISTING

<110>  Bejo Zaden BV
       Bejo Zaden BV

<120>  Lactuca sativa resistance to Bremia lactucae

<130>  BEJO-008-EP

<160>  7

<170>  PatentIn version 3.5

<210>  1
<211>  101
<212>  DNA
<213>  Lactuca

<220>
<221>  variation
<222>  (51)..(51)
<223>  T to C

<400>  1
gatagcyaaa accsaccaag ttrcggttca cccatagcta tttgtcatac tygttcttyt      60

taggggcttt ctggtttatg atgmatgcat ggatgycycr c                         101

<210>  2
<211>  101
<212>  DNA
<213>  Lactuca

<220>
<221>  variation
<222>  (51)..(51)
<223>  A to G

<400>  2
gtgagatgag agtttaaacc taaacarttt aagatgagca gttctacttg agatgactaa      60

gggtttggga acttasttgc aatgcatagt gagtcttgca c                         101

<210>  3
<211>  101
<212>  DNA
<213>  Lactuca

<220>
<221>  variation
<222>  (51)..(51)
<223>  T to C

<400>  3
gacccaccag gtgcaaatac cagcatttta ggacaatcaa tgatcccaac tatatccaat      60

gaaggccaka ggaatccatt catccccaag aagaaaccct c                         101

<210>  4
<211>  101
<212>  DNA
<213>  Lactuca

<220>
<221>  variation
<222>  (51)..(51)
<223>  A to C

<400>  4
gagtgycgtc tcatcacact atcaatctcc tcaattatat tgaaggcatt attccctacc      60

ttgtgcctwa tctttaratt raagcaacta wyracwtcac y                          101

<210>  5
<211>  101
<212>  DNA
<213>  Lactuca

<220>
<221>  variation
<222>  (51)..(51)
<223>  G to A

<400>  5
tatcattttg actttggatt taattataat tatggttata catcagttaa gaacatagta      60

gagtttacac ttgttgaaat cgctygatag taaaaatgat t                          101

<210>  6
<211>  101
<212>  DNA
<213>  Lactuca

<220>
<221>  variation
<222>  (51)..(51)
<223>  A to C

<220>
<221>  misc_feature
<222>  (78)..(78)
<223>  n is a, c, g, or t

<400>  6
agttcaatgg acttgagacg aggaaagrcc acaacctcct tygaagatgy atttgttgtt      60

tgctctccat attcatcntc ttccttcact atcactttca t                          101

<210>  7
<211>  101
<212>  DNA
<213>  Lactuca

<220>
<221>  variation
<222>  (51)..(51)
<223>  C to T

<400>  7
ggggagacgc cgtgygcttt tgtgaagctt aaagaagggt ttgagttarc cggtgatgat      60

ttgattktgt attgtmggaa aaatttaccg cattatatgg c                          101

## Description

### FIELD OF THE INVENTION

The present invention relates to a nucleic acid fragment comprising a resistance allele from *L. serriola* conferring broad spectrum resistance to *Bremia lactucae.* The invention further relates to a *Lactuca sativa* plant comprising said nucleic acid fragment and a method for producing said plant. Furthermore the invention also relates to cells, tissues, propagation material, seeds and progeny of said plants, comprising said nucleic acid fragment. The invention also relates to the use of marker sequences co-segregating with said resistance allele.

### BACKGROUND

*Bremia lactucae,* an oomycete, is the causal organism of downy mildew in lettuce (*Lactuca sativa* L.), and constitutes a major problem for lettuce production in both glass house and open field conditions. *Bremia lactucae* is an obligate parasite capable of infecting a lettuce plant in any growth stage from seedling to mature plant.

Downy mildew causes pale, angular, yellow areas bounded by veins on the upper leaf surfaces. Spore formation appears on the lower leaf surfaces as a white cottony-like fungal growth soon after initial symptom development. The lesions eventually turn brown, and they may enlarge and coalesce. These symptoms typically occur first on the lower leaves of the lettuce, but under ideal conditions may move into the upper leaves of the head. When the oomycete progresses to this degree, the head cannot be harvested. Less severe damage requires the removal of more leaves than usual, especially when the lettuce reaches its final destination. As such, every year this disease leads to millions of Euros costs due to lost lettuce crop throughout the world.

Breeding for lettuce resistant against *Bremia lactucae* has been based upon the identification and introgression of resistance genes (R-genes), known in lettuce as Dm-genes. When R-gene products of a lettuce plant recognize specific *Bremia* avirulence (Avr) gene products in a gene-for-gene interaction, downstream response pathways are triggered in the host plant. The result is an incompatible reaction associated with a hypersensitive cell death response by the host plant, thus providing race-specific resistance against *Bremia lactucae.*

WO 2009 111 627, WO 2011 003 783 and WO 2015 136 085 all disclose a cultivated *Lactuca sativa* plant comprising an introgression from wild *L. saligna* plant, genetically encoding for resistance to *Bremia lactucae.*

WO 2000 063 432 further discloses a cultivated *Lactuca sativa* plant comprising an introgression from a wild *L. virosa* plant, which confers a resistance to *Bremia lactucae.*

However, R-genes may be rendered ineffective soon after they are introduced due to the rapid genetic adaption of the pathogen. As new *Bremia lactucae* races or isolates emerge, their Avr genes have been altered in such a way thais allows the pathogen to evade recognition by the host and overcome race-specific resistance. Recognition of the altered Avr genes by existing R-genes is thus lost, and infection by newly emergent *Bremia lactucae* races or isolates can successfully be established resulting in disease.

For the lettuce plant, this means that the resistance provided by existing R-genes are broken by newly emerging races of the *Bremia lactucae* pathogen. Reestablishment of resistance in the plant can only occur however, if novel R-genes are introduced into the plant which are able to recognize other Avr genes. Thus breeders require novel resistance genes in order to keep producing resistant varieties. Consequently, there is a need in the field to provide new and alternative genes conferring *Bremia lactucae* resistance to enable breeders to develop novel lettuce cultivars that are effectively resistant to known *Bremia lactucae* races.

### SUMMARY OF THE INVENTION

Given the significant advantages of having alternative sources of resistance than those already present in the state of the art, the present invention relates to a nucleic acid fragment comprising a resistance allele from *L. serriola* conferring broad spectrum resistance to *Bremia lactucae* according to claim 1.

In a second aspect, the present invention relates to a cultivated lettuce (*Lactuca sativa* L.) plant comprising said nucleic acid fragment according to claim 4.

In a third aspect the present invention relates to a use of markers according to claim 11.

In a further aspect, the present invention relates to a method for the production of a cultivate lettuce (*Lactuca sativa* L.) plant according to claim 12.

### DETAILED DESCRIPTION OF THE INVENTION

The present invention relates to a nucleic acid fragment comprising a resistance allele conferring a broad spectrum resistance to *Bremia lactucae.* Furthermore the invention relates to a cultivated lettuce (*Lactuca sativa L*.) plant comprising said nucleic acid fragment, and cells, tissues, propagation material, seeds and progeny of said plant. The invention further relates to use of markers in marker assisted breeding and in the identification of the *Bremia resistance* trait.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

The term "lettuce" or "cultivated lettuce" or "cultivated *Lactuca sativa"* is understood within the scope of the invention to refer to plants of the species *Lactuca sativa* L. or seeds from which the plants can be grown, and parts of such plants. These are no longer in the natural state but have been developed by human intervention and for human use and/or consumption.

An "allele" is understood within the scope of the invention to refer to alternative or variant forms of various genetic units identical or associated with different forms of a gene or of any kind of identifiable genetic element, which are alternative in inheritance because they are situated at the same locus in homologous chromosomes. Such alternative or variant forms may be the result of single nucleotide polymorphism, insertions, inversions, translocations or deletions, or the consequence of gene regulation caused by, for example, by chemical or structural modification, transcription regulation or post-translational modification/regulation. In a diploid cell or organism, the two alleles of a given gene or genetic element typically occupy corresponding loci on a pair of homologous chromosomes. Alleles determine distinct traits that can be passed on from parents to offspring. The process by which alleles are transmitted was discovered by Gregor Mendel and formulated in what is known as Mendel's law of segregation.

An allele associated with an R-gene may comprise alternative or variant forms of various genetic units including those that are identical or associated with a single gene or multiple genes or their products or even a gene disrupting or controlled by a genetic factor contributing to the phenotype represented by the locus.

As used herein, the term "marker determinant" refers to an alternative or variant form of a genetic unit as defined herein above, when used as a marker to locate genetic loci containing alleles on a chromosome that contribute to variability of phenotypic traits.

As used herein, the term "breeding", and grammatical variants thereof, refer to any process that generates a progeny individual. Breeding can be sexual or asexual, or any combination thereof. Exemplary non-limiting types of breeding include crossings, selfings, doubled haploid derivative generation, and combinations thereof.

As used herein, the phrase "established breeding population" refers to a collection of potential breeding partners produced by and/or used as parents in a breeding program; e.g., a commercial breeding program. The members of the established breeding population are typically well-characterized genetically and/or phenotypically. For example, several phenotypic traits of interest might have been evaluated, e.g., under different environmental conditions, at multiple locations, and/or at different times. Alternatively or in addition, one or more genetic loci associated with expression of the phenotypic traits might have been identified and one or more of the members of the breeding population might have been genotyped with respect to the one or more genetic loci as well as with respect to one or more genetic markers that are associated with the one or more genetic loci.

A "SNP marker" as used herein is a single nucleotide base difference between two DNA sequences or individuals. SNPs can be categorized according to nucleotide substitutions either as transitions (C/T or G/A) or transversions (C/G, A/T, C/A or T/G). In practice, single base variants in cDNA (mRNA) are considered to be SNPs as are single base insertions and deletions (indels) in the genome. SNPs provide the ultimate/simplest form of molecular markers as a single nucleotide base is the smallest unit of inheritance, and thus they can provide maximum number of markers. SNPs occur very commonly in animals and plants. Typically, SNP frequencies are in a range of one SNP every 100-300 bp in plants. SNPs may present within coding sequences of genes, non-coding regions of genes or in the intergenic regions between genes at different frequencies in different chromosome regions.

As used herein, the phrase "diploid individual" refers to an individual that has two sets of chromosomes, typically one from each of its two parents. However, it is understood that in some embodiments a diploid individual can receive its "maternal" and "paternal" sets of chromosomes from the same single organism, such as when a plant is selfed to produce a subsequent generation of plants.

"Homozygous" is understood within the scope of the invention to refer to like alleles at one or more corresponding loci on homologous chromosomes.

"Heterozygous" is understood within the scope of the invention to refer to unlike alleles at one or more corresponding loci on homologous chromosomes.

"Backcrossing" is understood within the scope of the invention to refer to a process in which a hybrid progeny is repeatedly back crossed to one of the parents. Different recurrent parents may be used in subsequent backcrosses.

"Locus" is understood within the scope of the invention to refer to a region on a chromosome.

As used herein "marker locus" refers to a region on a chromosome, which comprises a nucleotide or a polynucleotide sequence that is present in an individual's genome and that is associated with one or more loci of interest, which may comprise a gene or any other genetic element or factor contributing to a trait. "Marker locus" also refers to a region on a chromosome, which comprises a polynucleotide sequence complementary to a genomic sequence, such as a sequence of a nucleic acid used as probes.

"Genetic linkage" is understood within the scope of the invention to refer to an association of characters in inheritance due to location of genes in proximity on the same chromosome, measured by percent recombination between loci (centi-Morgan, cM).

For the purpose of the present invention, the term "co-segregation" refers to the fact that the allele for the trait and the allele(s) for the marker(s) tend to be transmitted together because they are physically close together on the same chromosome (reduced recombination between them because of their physical proximity) resulting in a non-random association of their alleles as a result of their proximity on the same chromosome. "Co-segregation" also refers to the presence of two or more traits within a single plant of which at least one is known to be genetic and which cannot be readily explained by chance.

As used herein, the term "genetic architecture at the R-gene locus" refers to a genomic region which is statistically correlated to the phenotypic trait of interest and represents the underlying genetic basis of the phenotypic trait of interest.

As used herein, the phrases "sexually crossed" and "sexual reproduction" in the context of the presently disclosed subject matter refers to the fusion of gametes to produce progeny (e.g., by fertilization, such as to produce seed by pollination in plants). An "asexual cross" or "cross-fertilization" is in some embodiments fertilization of one individual by another (e.g., cross-pollination in plants). The term "selfing" refers in some embodiments to the production of seed by self-fertilization or self-pollination; i.e., pollen and ovule are from the same plant.

As used herein, the phrase "genetic marker" refers to a feature of an individual's genome (e.g., a nucleotide or a polynucleotide sequence that is present in an individual's genome) that is associated with one or more loci of interest. In some embodiments, a genetic marker is polymorphic in a population of interest, or the locus occupied by the polymorphism, depending on contest. Genetic markers include, for example, single nucleotide polymorphisms (SNPs), indels (i.e., insertions/deletions), simple sequence repeats (SSRs), restriction fragment length polymorphisms (RFLPs), random amplified polymorphic DNAs (RAPDs), cleaved amplified polymorphic sequence (CAPS) markers, Diversity Arrays Technology (DART) markers, and amplified fragment length polymorphism (AFLPs), among many other examples. Genetic markers can, for example, be used to locate genetic loci containing alleles on a chromosome that contribute to variability of phenotypic traits. The phrase "genetic marker" can also refer to a polynucleotide sequence complementary to a genomic sequence, such as a sequence of nuclei acids used as probes.

A genetic marker can be physically located in a position on a chromosome that is within or outside of the genetic locus with which it is associated (i.e., is intragenic or extragenic, respectively). Stated another way, whereas genetic markers are typically employed when the location on a chromosome of the gene or of a functional mutation, e.g. within a control element outside of a gene, that corresponds to the locus of interest has not been identified and there is a non-zero rate of recombination between the genetic marker and the locus of interest, the presently disclosed subject matter can also employ genetic markers that are physically within the boundaries of a genetic locus (e.g., inside a genomic sequence that corresponds to a gene such as, but not limited to a polymorphism within an intron or an exon of a gene).

"Microsatellite marker" or "SSRs (Simple Sequence Repeats) marker" is understood within the scope of the invention to refer to a type of genetic marker that consists of numerous repeats of short sequences of DNA bases, which are found at loci throughout the plant's genome and have a likelihood of being highly polymorphic.

As used herein, the term "genotype" refers to the genetic constitution of a cell or organism. An individual's "genotype for a set of genetic markers" includes the specific alleles, for one or more genetic marker loci, present in the individual's haplotype. As is known in the art, a genotype can relate to a single locus or to multiple loci, whether the loci are related or unrelated and/or are linked or unlinked. In some embodiments, an individual's genotype relates to one or more genes that are related in that the one or more of the genes are involved in the expression of a phenotype of interest (e.g., a quantitative or R-gene as defined herein). Thus, in some embodiment a genotypes comprises a summary of one or more alleles present within an individual at one or more genetic loci of a quantitative or R-gene. In some embodiments, a genotype is expressed in terms of a haplotype.

"Phenotype" is understood within the scope of the invention to refer to a distinguishable characteristic(s) of a genetically controlled trait.

As used herein, the term "phenotypic trait" refers to the appearance or other detectable characteristic of an individual, resulting from the interaction of its genome, proteome and/or metabolome with the environment.

As used herein, the term "germplasm" refers to the totality of the genotypes of a population or other group of individuals (e.g., a species). The term "germplasm" can also refer to plant material; e.g., a group of plants that act as a repository for various alleles. The phrase "adapted germplasm" refers to plant materials of proven genetic superiority; e.g., for a given environment or geographical area, while the phrases "non-adapted germplasm," "raw germplasm," and "exotic germplasm" refer to plant material of unknown or unproven genetic value; e.g., for a given environment or geographical area; as such, the phrase "non-adapted germplasm" refers in some embodiments to plant materials that are not part of an established breeding population and that do not have a known relationship to a member of the established breeding population.

As used herein, the terms "hybrid", "hybrid plant," and "hybrid progeny" refers to an individual produced from genetically different parents (e.g., a genetically heterozygous or mostly heterozygous individual is obtained).

As used herein, the phrase "single cross F1 hybrid" refers to an F1 hybrid produced from a cross between two inbred lines.

As used herein, the phrase "inbred line" refers to a genetically homozygous or nearly homozygous population. An inbred line, for example, can be derived through several cycles of brother/sister breedings or of selfing or in dihaploid production. In some embodiments, inbred lines breed true for one or more phenotypic traits of interest. An "inbred", "inbred individual", or "inbred progeny" is an individual sampled from an inbred line.

As used herein, the term "doubled haploid (DH) line", refers to stable inbred lines issued from anther culture. Some pollen grains (haploid) cultivated on specific medium and circumstances can develop plantlets containing n chromosomes. These plantlets are then "doubled" and contain 2n chromosomes. The progeny of these plantlets are named "doubled haploid" and are essentially not segregating any more (stable).

As used herein, the term "linkage", and grammatical variants thereof, refers to the tendency of alleles at different loci on the same chromosome to segregate together more often than would be expected by chance if their transmission were independent, in some embodiments as a consequence of their physical proximity.

"Linked markers" herein refers to molecular markers and/or phenotypic markers that co-segregate with the Bremia resistance phenotype or trait, such that by following the inheritance of the said molecular markers and/or phenotypic markers the inheritance of the trait can be followed.

As used herein, the phrase "nucleic acid" refers to any physical string of monomer units that can be corresponded to a string of nucleotides, including a polymer of nucleotides (e.g., a typical DNA, cDNA or RNA polymer), modified oligonucleotides (e.g., oligonucleotides comprising bases that are not typical to biological RNA or DNA, such as 2'-O-methylated oligonucleotides), and the like. In some embodiments, a nucleic acid can be single-stranded, double-stranded, multi-stranded, or combinations thereof. Unless otherwise indicated, a particular nucleic acid sequence of the presently disclosed subject matter optionally comprises or encodes complementary sequences, in addition to any sequence explicitly indicated.

As used herein, the term "resistance" refers to the ability of a plant to restrict the growth and development of a specified pathogen and/or the damage is causes when compared to susceptible plants under similar environmental conditions and pathogen pressure. Resistant plants may exhibit some disease symptoms or damage under pathogen pressure, e.g. fungal pathogen pressure such as *Bremia lactucae* pathogen pressure.

As used herein, the term "susceptibility" refers to the inability of a plant to adequately restrict the growth and development of a specified pathogen, e.g. fungal pathogen such as *Bremia lactucae.*

As used herein, the phrase *"Bremia* resistance" or "resistance to *Bremia* races" or *"Bremia* resistant plant" refers to the plants capability to resist colonization by the fungus *Bremia lactucae.* It further refers to the ability of a lettuce plant, variety, accession, line, etc. to restrict the growth and development of *Bremia lactucae,* isolates Bl:16 to Bl:36 as characterized and classified according to SEXTET code by IBEB (International *Bremia* Evaluation Board), and/or the damage that is caused by said oomycete, as compared to the ability to do so os a susceptible lettuce plant, variety accession, or line under similar environmental conditions and disease pressure.

As used herein, the term "plurality" refers to more than one. Thus, a "plurality of individuals" refers to at least two individuals, in some embodiments, the term plurality refers to more than half of the whole. For example, in some embodiments a "plurality of a population" refers to more than half the members of that population.

As used herein, the term "progeny" refers to the descendant(s) of a particular across. Typically, progeny result from breeding of two individuals, although some species (particularly some plants and hermaphroditic animals) can be selfed (i.e., the same plant acts as the donor of both male and female gametes). The descendant(s) can be, for example, of the F1, the F2, or any subsequent generation.

As used herein, the term "R-gene" refers to a phenotypic trait that is controlled by one or a few genes that exhibit major phenotypic effects. Because of this, R-genes are typically simply inherited. Examples in plants include, but are not limited to, flower color, fruit color, and several known disease resistances.

"Marker-assisted selection" is understood within the scope of the invention to refer to e.g. the use of genetic markers to detect one or more nucleic acids from the plant, where the nucleic acid is associated with a desired trait to identify plants that carry genes for desirable (or undesirable) traits, so that those plants can be used (or avoided) in a selective breeding program.

"PCR (polymerase chain reaction)" is understood within the scope of the invention to refer to a method of producing relatively large amounts of specific regions of DNA or subset(s) of the genome, thereby making possible various analyses that are based on those regions.

"PCR primer" is understood within the scope of the invention to refer to relatively short fragments of single-stranded DNA used in the PCR amplification of specific regions of DNA.

"Polymorphism" is understood within the scope of the invention to refer to the presence in a population of two or more different forms of a gene, genetic marker, or inherited trait or a gene product obtainable, for example, through alternative splicing, DNA methylation, etc.

"Selective breeding" is understood within the scope of the invention to refer to a program of breeding that uses plants that possess or display desirable traits as parents.

"Tester" plant is understood within the scope of the invention to refer to a plant of the genus *Lactuca* used to characterize genetically a trait in a plant to be tested. Typically, the plant to be tested is crossed with a "tester" plant and the segregation ratio of the trait in the progeny of the cross is scored.

"Probe" as used herein refers to a group of atoms or molecules which is capable of recognizing and binding to a specific target molecule or cellular structure and thus allowing detection of the target molecule or structure. Particularly, "probe" refers to a labelled DNA or RNA sequence which can be used to detect the presence of and to quantitate a complementary sequence by molecular hybridization.

"Sequence homology" or "sequence identity" is used herein interchangeably. The terms "identical" or "percent identity" in the context of two or more nucleic acid or protein sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. If two sequences which are to be compared with each other differ in length, sequence identity preferably relates to the percentage of the nucleotide residues of the shorter sequence which are identical with the nucleotide residues of the longer sequence. When using Bestfit or another sequence alignment program to determine whether a particular sequence has for instance 95% identity with a reference sequence of the present invention, the parameters are preferably adjusted such that the percentage of identity is calculated over the entire length of the reference sequence and that homology gaps of up to 5% of the total number of the nucleotides in the reference sequence are permitted. When using Bestfit, the so-called optional parameters are preferably left at their preset ("default") values. The deviations appearing in the comparison between a given sequence and the above-described sequences of the invention may be caused for instance by addition, deletion, substitution, insertion or recombination. Such a sequence comparison may for instance be carried out by a Smith-Waterman BLAST alignment.

A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant.

"Plant cell culture" means cultures of plant units such as, for example, protoplasts, cell culture cells, cells in plant tissues, pollen, pollen tubes, ovules, embryo sacs, microspores, zygotes and embryos at various stages of development.

"Plant materials" or "plant materials obtainable from a plant" refers to leaves, stems, roots, flowers or flower parts, fruits, pollen, egg cells, zygotes, seeds, cuttings, cell or tissue cultures, or any other part or product of a plant.

A "plant organ" is a distinct and visibly structured and differentiated part of a plant such as a root, stem, leaf, flower bud, or embryo.

"Plant tissue" as used herein means a group of plant cells organized into a structural and functional unit. Any tissue of a plant or plant parts in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

The terms "race" or "races" refer to any inbreeding group, including taxonomic subgroups such as subspecies, taxonomically subordinate to a species and superordinate to a subrace and marked by a pre-determined profile of latent factors of hereditary traits.

The cultivated lettuce, *Lactuca sativa* or *Lactuca sativa* L., is a temperate annual or biennial plant most often grown as a leaf vegetable. Lettuce belongs to the aster or sunflower family Asteraceae or Compositae. Other members of this family include endive, chicory, artichoke, sunflower and safflower. *L. sativa* is closely related to common wild lettuce or prickly lettuce, *L. serriola,* and is less closely related to two other wild lettuces *L. saligna* and *L. virosa.*

The lettuce *Lactuca sativa* has been genetically characterized by whole-genome shotgun strategy resulting into a genome assembly of nine chromosomal linkage groups. The resulting high-quality reference genome reveals the plant genetic resources of lettuce and facilitates map-based cloning of agricultural important genes from lettuce that is required for crop improvement using genome editing, particularly those involved in resistance to *Bremia lactucae.*

The present invention relates to a nucleic acid fragment comprising a resistance allele from *L. serriola* which confers a broad spectrum resistance to *Bremia lactucae.* Moreover, the resistance allele may encode for protein(s) correlated with the improved resistance, particularly a general, race non-specific resistance to *Bremia lactucae* in terms of races known as of the filing data of the present application, particularly to (at least) *Bremia* races or isolates Bl:16 to BI:36 characterized and classified according to the SEXTET code by IBEB (International *Bremia* Evolution Board).

A person skilled in the art will appreciate that the resistance allele from *L. serriola* conferring resistance to *Bremia lactucae* has been introduced into a cultivated lettuce *(Lactuca sativa* L.) plant. Since *Lactuca serriola* is resistant to all downy mildew races *L. serriola* is an excellent source for genetically-based resistance to the downy mildew pathogen, *Bremia lactucae,* in lettuce. Said resistance allele from *L. serriola* encoding for resistance to *Bremia lactucae* is different than the ones originating from *L. saligna* and *L. virosa* and more durable than typical race-specific resistance. Therefore, the provision of a nucleic acid fragment comprising the resistance allele from *L. serriola* conferring broad spectrum resistance to *Bremia lactucae* is an environmentally friendly alternative for the use of pesticides and will contribute to successful integrated pest management programs.

Characterization and evaluation of plant genetic resources are prerequisites for the efficient use of the material, be it through methods of crossing, selecting and breeding or other techniques. Until recently, most of the characterization and evaluation of plant genetic resources have been based on recordings of either qualitative and/or quantitative morphological characters. During the past decade or so, more and more emphasis has been placed on biochemical characterization and more recently on the use of molecular techniques. Molecular genetic characterization has several advantages, such as no environmental influences, any plant part from any growth stage can be used, there is no limit on numbers for analysis, only small amounts of material are required, and since DNA is highly stable, even dry samples can be used.

Molecular or genetic markers can aid in the development of new novel traits that can be put into mass production. These novel traits can be identified using molecular markers and maps. An identifiable marker may help follow particular traits of interest, like resistance, when crossing between different species, with the aim of transferring particular traits to the offspring.

In a first embodiment, the present invention relates to a nucleic acid fragment comprising a resistance allele from *L. serriola* which confers a broad spectrum resistance to *Bremia lactucae,* wherein said resistance allele co-segregates with one or more marker sequences chosen from Seq ID No. 1, Seq ID No. 2, Seq ID No. 3, Seq ID No. 4, Seq ID No. 5, Seq ID No. 6, Seq ID No. 7, and/or any *L. serriola* genome specific marker in between Seq ID No. 1 and Seq ID No. 7. The nucleic acid fragment according to the current invention will preferably comprise a *Bremia* resistance allele, linked to a genetic determinant and obtainable from the genome of a wild *Lactuca* plant, particularly from *L. serriola.* In a specific embodiment of the invention, the resistance to *Bremia* is a general, race non-specific resistance. In a further embodiment it is aimed to reduce linkage drag or co-expression of agronomical undesirable traits such as, for example dwarfism, to a minimum.

In a preferred embodiment the invention relates to the resistance allele from *L*. *serriola,* which confers a broad spectrum resistance to *Bremia lactucae.* In an embodiment said resistance allele co-segregates with one or more marker sequences chosen from Seq ID No. 1, Seq ID No. 2, Seq ID No. 3, Seq ID No. 4, Seq ID No. 5, Seq ID No. 6, Seq ID No. 7, and/or any *L*. *serriola* genome specific marker in between Seq ID No. 1 and Seq ID No. 7. In a specific embodiment the resistance allele co-segregates with all marker sequences chosen from Seq ID No. 1, Seq ID No. 2, Seq ID No. 3, Seq ID No. 4, Seq ID No. 5, Seq ID No. 6, and Seq ID No. 7. In another specific embodiment, the resistance allele co-segregates with one marker sequence, preferably at least 2 marker sequences, preferably at least 3 marker sequences, more preferably at least 4 marker sequences, more preferably at least 5 marker sequences, more preferably at least 6 marker sequences chosen from Seq ID No. 1, Seq ID No. 2, Seq ID No. 3, Seq ID No. 4, Seq ID No. 5, Seq ID No. 6, and/or Seq ID No. 7.

In a preferred embodiment marker sequence Seq ID No. 1 co-segregates with the resistance allele. In another preferred embodiment one or more marker sequences selected from the group consisting of Seq ID No. 2, Seq ID No. 3, Seq ID No. 4, Seq ID No. 5, Seq ID No. 6 and/or Seq ID No. 7 co-segregate with the resistance allele.

These marker sequences contain a single nucleotide polymorphism in their sequence and are called SNP markers. In one embodiment said markers are used for plant breeding. Preferably the markers are tightly linked to the target resistance allele. Preferably the markers have less than 10 cM, more preferably less than 5 cM genetic distance, even more preferably flanking the gene of interest and ideally the markers being intragenic.

In a further specific embodiment of the invention the nucleic acid fragment is introduced into a cultivated lettuce (*Lactuca sativa* L.) plant, wherein the nucleic acid fragment comprising the resistance allele is located on chromosome 2, preferably between 6.9 and 11.8 Mbp of said chromosome 2. Preferably said fragment is located between 7.4 and 11.3 Mbp, more preferably between 7.8 and 10.8 Mbp, more preferably between 8 and 10.6 Mbp, even more preferably between 8.4 and 10.5 Mbp on chromosome 2.

The introgression fragment may be large, e.g. half of a chromosome, but is in particular smaller, such as 15 Mb or less, such as about 10 Mbp or less, about 9 Mbp or less, about 8 Mbp or less, about 7 Mbp or less, about 6 Mbp or less, about 5 Mbp or less, about 4 Mbp or less, about 3 Mbp or less, about 2 Mbp or less. It is understood in current invention that the fragment never includes the entire chromosome, but only part of the chromosome.

The invention also relates to the use of the nucleic acid fragment of the invention or any fragment of said nucleic acid as a screening tool for identifying a new ligand peptide interacting with the protein encoded by nucleic acid of the invention. Methods of the art are well known for the identification of ligand-protein interaction like, for example, the yeast two-hybrid system (Fields and Song, 1989) or by immunoprecipitation.

The invention is also encompassing a method for modifying the nucleic acid of the invention to improve the function of the protein encoded by said nucleic acid fragment in order to improve the resistance to *Bremia lactucae.* Method of the art related to the modification of the genomic DNA or "gene editing" are well-known like, for example TALENs (WO 2011 072 246) or CRISPR/Cas9.

In a preferred embodiment, the nucleic acid fragment comprising said resistance allele is as found in the genome of a cultivated lettuce (*Lactuca sativa* L.) plant grown from seeds of which a representative sample was deposited under NCIMB Accession No. NCIMB 43292.

In accordance with the invention, a novel variety may be created by crossing *Bremia lactucae* resistant *L. serriola* with *Bremia lactucae* susceptible *L. sativa,* followed by generations of selection as desired and inbreeding for development of uniform lines comprising nucleic acid fragment of current invention.

For development of a uniform line, often five or more generations of selfing and selection are typically involved. Uniform lines of new varieties may also be developed by way of doubled-haploids. This technique allows the creation of true breeding lines without the need for multiple generations of selfing and selection. In this manner true breeding lines can be produced in as little as one generation. Haploid embryos may be produced from microspores, pollen, anther cultures, or ovary cultures. The haploid embryos may then be doubled autonomously, or by chemical treatments (e.g. colchicine treatment). Alternatively, haploid embryos may be grown into haploid plants and treated to induce chromosome doubling. In either case, fertile homozygous plants are obtained. In accordance with the invention, any of such techniques may be used in connection with a Downy Mildew resistant line of the present invention and progeny thereof to achieve a homozygous line.

Backcrossing can also be used to improve an inbred plant. Backcrossing transfers a specific desirable trait, such as Downy Mildew resistance, from one inbred or non-inbred source to an inbred that lacks that trait. This can be accomplished, for example, by first crossing a superior inbred (A) (recurrent parent) to a donor inbred (non-recurrent parent), which carries the appropriate locus or loci for the trait in question. The progeny of this cross are then mated back to the superior recurrent parent (A) followed by selection in the resultant progeny for the desired trait to be transferred from the non-recurrent parent. After five or more backcross generations with selection for the desired trait, the progeny is heterozygous for loci controlling the characteristic being transferred, but are like the superior parent for most or almost all other loci. The last backcross generation would be selfed to give pure breeding progeny for the trait being transferred.

The selection of a suitable recurrent parent is an important step for a successful backcrossing procedure. The goal of a backcross protocol is to alter or substitute a single trait or characteristic in the original variety. To accomplish this, a single locus of the recurrent variety is modified or substituted with the desired locus from the nonrecurrent parent, while retaining essentially all of the rest of the desired genetic, and therefore the desired physiological and morphological constitution of the original variety. The choice of the particular non-recurrent parent will depend on the purpose of the backcross; one of the major purposes is to add some commercially desirable trait to the plant. The exact backcrossing protocol will depend on the characteristic or trait being altered to determine an appropriate testing protocol. Although backcrossing methods are simplified when the characteristic being transferred is a dominant allele, a recessive allele may also be transferred. In this instance it may be necessary to introduce a test of the progeny to determine if the desired characteristic has been successfully transferred.

A preferred embodiment of current invention comprises a cultivated lettuce (*Lactuca sativa* L.) plant, wherein said plant comprises the nucleic acid fragment from *L. serriola* comprising the resistance allele which confers a broad spectrum resistance to *Bremia lactucae,* wherein the resistance allele is located on chromosome 2 and co-segregates with one or more marker sequences chosen from Seq ID No. 1, Seq ID No. 2, Seq ID No. 3, Seq ID No. 4, Seq ID No. 5, Seq ID No. 6, and Seq ID No. 7. In another preferred embodiment the cultivated lettuce (*Lactuca sativa* L.) confers a broad spectrum resistance to all *Bremia lactucae* races known as of the filing date of the present application, more preferably to *Bremia lactucae* races Bl:16 to BI:36.

In a further embodiment, the present invention also contemplates a plant according to any of the preceding embodiments wherein the presence of the *Bremia lactucae* resistance allele is characterized by at least one marker on chromosome 2 that is statistically correlated and thus genetically linked to the *Bremia lactucae* resistance trait.

In a further embodiment, a cultivated *L. sativa* plant is provided comprising the resistance allele, at least one allele in the *L. sativa* genome contributes to resistance to *Bremia lactucae,* which is genetically linked to at least one marker sequence, which co-segregates with the *Bremia lactucae* resistance trait and that can be identified by at least one PCR oligonucleotide primer or by any other marker on the chromosome that is statistically correlated and thus genetically linked to the *Bremia lactucae* resistance trait.

In one embodiment, said resistance allele in the *L. sativa* genome conferring *Bremia lactucae* resistance, is obtainable from a plant which has the genetic background of *Lactuca sativa* line X2406904, particularly from a plant which has the genetic background or architecture at the R-gene locus of *L. sativa* line X2406904, but especially from a representative seed of which is deposited at the National Collection of Industrial Food and Marine Bacteria under NCIMB Accession No. NCIMB 43292, or from a progeny or an ancestor thereof comprising said resistance allele.

In a further embodiment, the present invention also relates to a plant according to any of the preceding embodiments, wherein said plant is a doubled haploid or a hybrid. In another embodiment, a plant according to any of the preceding embodiments is also contemplated, wherein said plant is male sterile. In a preferred embodiment, the *L. sativa* plant is heterozygous for the *Bremia lactucae* resistance trait. In another preferred embodiment of the invention, the *L. sativa* plant is homozygous for the *Bremia lactucae* resistance trait.

Selection of lettuce plants for breeding is not necessarily dependent on the phenotype of a plant and instead can be based on molecular investigations. For example, one can utilize a suitable genetic marker which is closely genetically linked to a trait of interest, like resistance to *Bremia lactucae.* One of these markers can be used to identify the presence or absence of a trait in the offspring of a particular cross, and can be used in selection of progeny for continued breeding. This technique is commonly referred to as marker assisted selection. Therefore the introgression of the nucleic acid fragment from *L. serriola* into the cultivated *Lactuca sativa* plant is not exclusively obtained by an essentially biological method as molecular techniques are applied. Any other type of genetic marker or other assay which is able to identify the relative presence or absence of a trait of interest in a plant can also be useful for breeding purposes.

Markers of the invention may contain one or more Single Nucleotide Polymorphism or SNP identified between two different susceptible and resistant genomes. It is also possible to identify sequence deletion/insertion (INDEL) polymorphism.

Any method known in the art may be used in the art to assess the presence or absence of a SNP. Some suitable methods include, but are not limited to, sequencing, hybridization assays, polymerase chain reaction (PCR), ligase chain reaction (LCR), and genotyping-by-sequence (GBS), or combinations thereof. Different PCR-based methods are available to the person skilled of the art. One can use the real-time PCR method or the KASP (Kompetitive allele specific PCR) method from KBioscience.

In a preferred embodiment marker sequences Seq ID No. 1, Seq ID No. 2, Seq ID No. 3, Seq ID No. 4, Seq ID No. 5, Seq ID No. 6, Seq ID No. 7, and/or any *L. serriola* genome specific marker in between Seq ID No. 1 and Seq ID No. 7 are used to identify the presence or absence of the nucleic acid fragment comprising the resistance allele of current invention. In a further embodiment said marker sequences are used to identify and develop other marker sequences co-segregating with the nucleic acid fragment of current invention. In particular the marker sequences of the invention can also be used as a probe to identify and isolate orthologs of the nucleic acid fragment in other plant species.

In a further embodiment, the present invention relates also to the use of some or all of these marker sequences for diagnostic selection of a lettuce conferring resistance to *Bremia* in *L. sativa.*

This molecular technique using marker sequences allows plant breeders to save time as compared to often laborious phytopathological tests. Testing with markers can be performed at all stages of plant development, which is not always the case for a reliable disease test. Moreover, the genetic tests for the presence of markers are not dependent on field circumstances which can or cannot promote the development of the disease and are generally less susceptible to experimental variation as compared to pathological test making them more reliable.

Any method known in the art may be used to assess the presence or absence of a nucleic acid fragment in the genome of a plant. Some suitable methods include, but are not limited to, sequencing, hybridization assays, polymerase chain reaction (PCR), ligase chain reaction (LCR).

Methods for marker assisted selection are of particular utility for introgression of given traits. Types of genetic markers that could be used in accordance with the invention include, but are not necessarily limited to, Simple Sequence Length Polymorphisms (SSLPs), Cleaved Amplified Polymorphic Sequences (CAPs), Randomly Amplified Polymorphic DNAs (RAPDs), DNA Amplification Fingerprinting (DAF), Sequence Characterized Amplified Regions (SCARs), Arbitrary Primed Polymerase Chain Reaction (AP-PCR), Amplified Fragment Length Polymorphisms (AFLPs), and Single Nucleotide Polymorphisms (SNPs).

In accordance with the invention, a novel variety may also be created by introducing the nucleic acid fragment from *L. serriola* comprising the resistance allele against *Bremia lactucae* into the genome of a host plant, propagation material, tissue and/or cell of *Lactuca sativa* L. by molecular techniques.

The skilled person will readily understand that introgression is however not the sole manner in which the nucleic acid fragment comprising the resistance allele or the resistance allele can be introduced in the genome of a cultivated lettuce plant. Other methods may for instance involve the use of transgenic techniques, such as haploid cell fusion, plant transformation and the like. The introduction of said nucleic acid fragment may therefore also be performed by *in vitro* culture techniques, by protoplast fusion, by transformation or by a doubled haploid technique. Often, such techniques are not performed with intact plants but with propagation material, cells and/or tissues of said plants.

In a preferred embodiment a cell or tissue from a cultivated lettuce (*Lactuca sativa* L.) plant of current invention has a broad spectrum resistance to *Bremia lactucae* and said cell and tissue are suitable for culturing. In a specific embodiment, said nucleic acid fragment is stably integrated within the genome of said cell or tissue. This embodiment is particularly interesting for *Lactuca sativa* plant cells or tissues. Stable integration within the genome means that the expression of the allele encoding for resistance can be transmitted to the progeny of said plant cell or tissue upon division.

It is reminded that a whole plant can be regenerated from a single transformed plant cell, providing a transgenic plant or a part thereof. The regeneration can proceed by known methods. The seeds which grow by fertilization from this plant, also contain this transgene in their genome.

In another preferred embodiment propagation material is derived from a cultivated lettuce plant (*Lactuca sativa* L.) of current invention, wherein said fragment has a broad spectrum resistance to *Bremia lactucae,* and wherein said material is selected from the group consisting of microspores, pollen, ovaries, ovules, embryos, embryo sacs, egg cells, cuttings, roots, root tips, root stocks, shoots, hypocotyls, cotyledons, stems, anthers, leaves, petioles, flowers, anthers, seeds, meristematic cells, protoplasts, and cells and said propagation material is capable of growing into a cultivated lettuce (*Lactuca sativa* L.) plant.

In order to obtain a cultivated lettuce (*Lactuca sativa* L.) plant of the invention such methods may optionally include a further step of growing said cell, tissue, propagation material into a lettuce plant. Generally this will involve production of said plants from the cell, tissue or propagation material transformed with heterologous DNA and, optionally, biologically replicating said cell, tissue, propagation material or both.

Resistance and other useful traits can be introduced into a plant by genetic transformation techniques. Vectors may be employed for the genetic transformation of a nucleic acid fragment into *L. sativa* cell. A vector, such as a plasmid, can thus be used for transforming lettuce cells. The construction of vectors for transformation of lettuce cells is within the capability of one skilled in the art following standard techniques.

Where a naked nucleic acid fragment introduction method is used, then the vector can be the minimal nucleic acid fragment necessary to confer the desired phenotype, without the need for additional sequences.

Vectors used for the transformation of lettuce cells are not limited so long as the vector can express an inserted fragment in the cells. For example, vectors comprising promoters for constitutive gene expression in lettuce cells (e.g., cauliflower mosaic virus 35S promoter) and promoters inducible by exogenous stimuli can be used. Possible vectors include pBI binary vector, the Ti plasmid vectors, shuttle vectors designed merely to maximally yield high numbers of copies, episomal vectors containing minimal sequences necessary for ultimate replication once transformation has occurred, transposon vectors, including the possibility of RNA forms of the gene sequences. The selection of vectors and methods to construct them are commonly known to persons of ordinary skill in the art and are described in general technical references.

The "lettuce cell" into which the vector is to be introduced includes various forms of lettuce cells, such as cultured cell suspensions, protoplasts, leaf sections, and callus.

A vector can be introduced into lettuce cells by known methods, such as the polyethylene glycol method, polycation method, electroporation, *Agrobacterium-*mediated transfer, particle bombardment and direct DNA uptake by protoplasts.

To effect transformation by electroporation, one may employ either friable tissues, such as a suspension culture of cells or embryogenic callus or alternatively one may transform immature embryos or other organized tissue directly. In this technique, one would partially degrade the cell walls of the chosen cells by exposing them to pectin-degrading enzymes (pectolyases) or mechanically wound tissues in a controlled manner.

A particularly efficient method for delivering transforming DNA segments to plant cells is microprojectile bombardment. In this method, particles are coated with nucleic acids and delivered into cells by a propelling force. Exemplary particles include those comprised of tungsten, platinum, and preferably, gold. For the bombardment, cells in suspension are concentrated on filters or solid culture medium. Alternatively, immature embryos or other target cells may be arranged on solid culture medium. The cells to be bombarded are positioned at an appropriate distance below the macroprojectile stopping plate.

Microprojectile bombardment techniques are widely applicable, and may be used to transform virtually any plant species.

*Agrobacterium-mediated* transfer is another widely applicable system for introducing gene loci into plant cells. An advantage of the technique is that DNA can be introduced into whole plant tissues, thereby bypassing the need for regeneration of an intact plant from a protoplast. Modern *Agrobacterium* transformation vectors are capable of replication in *E. coli* as well as *Agrobacterium,* allowing for convenient manipulations. Moreover, recent technological advances in vectors for *Agrobacterium-mediated* gene transfer have improved the arrangement of genes and restriction sites in the vectors to facilitate the construction of vectors capable of expressing various polypeptide coding genes. The vectors described have convenient multi-linker regions flanked by a promoter and a polyadenylation site for direct expression of inserted polypeptide coding genes. Additionally, *Agrobacterium* containing both armed and disarmed Ti (Tumor inducing) genes can be used for transformation.

In those plant strains where *Agrobacterium-mediated* transformation is efficient, it is the method of choice because of the facile and defined nature of the gene locus transfer. The use of *Agrobacterium-mediated* plant integrating vectors to introduce DNA into plant cells is well known in the art.

Transformation of plant protoplasts also can be achieved using methods based on calcium phosphate precipitation, polyethylene glycol treatment, electroporation, and combinations of these treatments.

The nucleic acid fragment can then be used in a construct under an operably linked heterologous promoter. As used herein, heterologous promoter means a promoter which does not originate from the same species from which the nucleic acid was derived, or the promoter is from the same species from which the nucleic acid was derived but has been modified to obtain a sequence different from the native sequence. Operably linked means that there is a functional linkage between the regulatory element (the promoter) and the nucleic acid to allow the expression of the nucleic acid. Both elements can be separated by sequence that can enhance the expression of the nucleic acid like introns.

In a preferred embodiment, the nucleic acid fragment of the invention is cloned downstream of a heterologous promoter functional in a plant cell. A promoter "active in plants" is a promoter that is able to drive expression of a gene operably linked thereto in a plant cell. Although some promoters may have the same pattern of regulation when there are used in different species, it is often preferable to use monocotyledonous promoters in monocotyledons and dicotyledonous promoters in dicotyledonous plants. Preferably, said construct is under the control of a constitutive promoter. Examples of constitutive promoters useful for expression include the 35S promoter or the 19S promoter, the rice actin promoter, the pCRV promoter, the CVMV promoter, the ubiquitin 1 promoter of maize, the regulatory sequences of the T-DNA of *Agrobacterium tumefaciens,* including mannopine synthase, nopaline synthase, octopine synthase. More preferably the promoters used in the invention are those expressed during seed development.

Other suitable promoters could be used. It could be an inducible promoter, a developmentally regulated promoted or a tissue-specific promoter such as a leaf-specific promoter, a seed-specific, a BETL specific promoter and the like. Numerous tissue- specific promoters are described in the literature and any one of them can be used. One can cite the promoters disclosed in US 20130024998.

A number of promoters have utility for plant gene expression for any gene of interest including but not limited to selectable markers, scorable markers, genes for pest tolerance, disease resistance, nutritional enhancements and any other gene of agronomic interest. Examples of constitutive promoters useful for lettuce plant gene expression include, but are not limited to, the cauliflower mosaic virus (CaMV) P-35S promoter, which confers constitutive, high-level expression in most plant tissues, including monocots; a tandemly duplicated version of the CaMV 35S promoter, the enhanced 35S promoter (P-e35S) the nopaline synthase promoter, the octopine synthase promoter; and the figwort mosaic virus (P-FMV) promoter and an enhanced version of the FMV promoter (P-eFMV) where the promoter sequence of P-FMV is duplicated in tandem, the cauliflower mosaic virus 19S promoter, a sugarcane bacilliform virus promoter, a commelina yellow mottle virus promoter, and other plant DNA virus promoters known to express in plant cells.

A variety of plant gene promoters that are regulated in response to environmental, hormonal, chemical, and/or developmental signals can be used for expression of an operably linked gene in plant cells, including promoters regulated by (1) heat, (2) light, (3) hormones, (4) wounding, or (5) chemicals. It may also be advantageous to employ organ-specific promoters.

Efficient genome engineering in plants can be enabled by introducing targeted double-strand breaks (DSBs) in a DNA sequence to be modified. The DSBs activate cellular DNA repair pathways, which can be harnessed to achieve desired DNA sequence modifications near the break site. Targeted DSBs can be introduced using sequence-specific nucleases (SSNs), a specialized class of proteins that includes transcription activator-liked (TAL) effector endonucleases, zinc-finger nucleases (ZFNs), and homing endonucleases (HEs). Recognition of a specific DNA sequence is achieved through interaction with specific amino acids encoded by the SSNs. Prior to the development of TAL effector endonucleases, a challenge of engineering SSNs was the unpredictable context dependencies between amino acids that bind to DNA sequence. While TAL effector endonucleases greatly alleviated this difficulty, their large size (on average, each TAL effector endonuclease monomer contains 2.5-3 kb of coding sequence) and repetitive nature may hinder their use in applications where vector size and stability is a concern.

Also the CRISPR/Cas system can be used for plant genome engineering. The CRISPR/Cas system provides a relatively simple, effective tool for generating modifications in genomic DNA at selected sites. CRISPR/Cas systems can be used to create targeted DSBs or single-strand breaks, and can be used for, without limitation, targeted mutagenesis, gene targeting, gene replacement, targeted deletions, targeted inversions, targeted translocations, targeted insertions, and multiplexed genome modification through multiple DSBs in a single cell directed by co-expression of multiple targeting RNAs. This technology can be used to accelerate the rate of functional genetic studies in plants, and to engineer plants with improved characteristics, including enhanced nutritional quality, increased resistance to disease and stress, and heightened production of commercially valuable compounds. Materials and methods for gene targeting using Clustered Regularly Interspersed Short Palindromic Repeats/CRISPR-associated (CRISPR/Cas) systems are provided in WO 2014 144 155.

One embodiment of current invention also relates to a method for generating a cultivated lettuce (*Lactuca sativa* L.) plant, wherein the method comprises an introgression from any *Lactuca* L. species on chromosome 2 comprising a resistance allele from any *Lactuca* L. species which confers a broad spectrum resistance to *Bremia lactucae* races BI:16 to BI:36, characterized in that said, introgression comprises the sequence of Seq ID No. 1, Seq ID No. 2, Seq ID No. 3, Seq ID No. 4, Seq ID No. 5, Seq ID No. 6, and/or Seq ID No. 7 by using tissue culture.

One embodiment of current invention also relates to a method for generating a cultivated lettuce (*Lactuca sativa* L.) plant, wherein the method comprises an introgression from *L. serriola* on chromosome 2 comprising a resistance allele from *L. serriola* which confers a broad spectrum resistance to *Bremia lactucae* races Bl:16 to BI:36, wherein said introgression comprises the sequence of Seq ID No. 1, Seq ID No. 2, Seq ID No. 3, Seq ID No. 4, Seq ID No. 5, Seq ID No. 6, and/or Seq ID No. 7 by using tissue culture.

In an embodiment the method for generating a cultivate lettuce (*Lactuca sativa* L.) plant may, without limitation, be executed by using a tissue culture. Said tissue culture preferably comprises cells, tissues or propagation material according to previous embodiments of the invention.

Another preferred embodiment of the invention relates to a method for production of a cultivated lettuce plant (*Lactuca sativa* L.) that comprises an introgression from any *Lactuca* L. species on chromosome 2 comprising a resistance allele from any *Lactuca* L. species which confers a broad spectrum resistance to *Bremia lactucae* races Bl:16 to Bl:36, characterized in that said, introgression comprises the sequence of Seq ID No. 1, Seq ID No. 2, Seq ID No. 3, Seq ID No. 4, Seq ID No. 5, Seq ID No. 6, and/or Seq ID No. 7 by using vegetative reproduction.

Another preferred embodiment of the invention relates to a method for production of a cultivated lettuce plant (*Lactuca sativa* L.) that comprises an introgression from *L. serriola* on chromosome 2 comprising a resistance allele from *L. serriola* which confers a broad spectrum resistance to *Bremia lactucae* races BI:16 to BI:36, characterized in that said, introgression comprises the sequence of Seq ID No. 1, Seq ID No. 2, Seq ID No. 3, Seq ID No. 4, Seq ID No. 5, Seq ID No. 6, and/or Seq ID No. 7 by using vegetative reproduction.

### SEED DEPOSIT

The cultivar of current invention is deposited with the National Collection of Industrial Food and Marine Bacteria, now at Bucksburn, Aberdeen, AB21 9YAY, Scotland, as seeds under the NCIMB Accession No. NCIMB 43292, on November 27, 2018.

### SEQUENCE LISTING

A sequence listing is provided containing 7 marker sequences and which is hereby incorporated by reference in its entirety. The 7 marker sequences originating from a *Lactuca* sp. are listed in Table 1. The marker sequences each contain a SNP at location 51, which is marked in bold in the nucleotide sequence. The SNP nucleotide is depicted in the final column, wherein the first SNP nucleotide is present on the resistant allele, whereas the second SNP nucleotide is present on the susceptible allele.

**Table 1:**

| SEQ ID No. | Genetic position on chromosome 2 (bp) | Nucleotide sequence (5' - 3') | SNP nucleotide at location 51 |
|---|---|---|---|
| SEQ ID No. 1 | 8414761 | GATAGCYAAAACCSACCAAGTTRCGGTTCACCCATAGCTATTTGTCATACTYGTTCTTYTT AGGGGCTTTCTGGTTTATGATGMATGCATGGATGYCYCRC | T to C |
| SEQ ID No. 2 | 10167266 | GTGAGATGAGAGTTTAAACCTAAACARTTTAAGATGAGCAGTTCTACTTGAGATGACTAA GGGTTTGGGAACTTASTTGCAATGCATAGTGAGTCTTGCAC | A to G |
| SEQ ID No. 3 | 10161129 | GACCCACCAGGTGCAAATACCAGCATTTTAGGACAATCAATGATCCCAACTATATCCAAT GAAGGCCAKAGGAATCCATTCATCCCCAAGAAGAAACCCTC | T to C |
| SEQ ID No. 4 | 10165216 | GAGTGYCGTCTCATCACACTATCAATCTCCTCAATTATATTGAAGGCATTATTCCCTACCT TGTGCCTWATCTTTARATTRAAGCAACTAWYRACWTCACY | A to C |
| SEQ ID No. 5 | 10166191 | TATCATTTTGACTTTGGATTTAATTATAATTATGGTTATACATCAGTTAAGAACATAGTAG AGTTTACACTTGTTGAAATCGCTYGATAGTAAAAATGATT | G to A |
| SEQ ID No. 6 | 10280540 | AGTTCAATGGACTTGAGACGAGGAAAGRCCACAACCTCCTTYGAAGATGYATTTGTTGTT TGCTCTCCATATTCATCNTCTTCCTTCACTATCACTTTCAT | A to C |
| SEQ ID No. 7 | 10410045 | GGGGAGACGCCGTGYGCTTTTGTGAAGCTTAAAGAAGGGTTTGAGTTARCCGGTGATGA TTTGATTKTGTATTGTMGGAAAAATTTACCGCATTATATGGC | C to T |

### EXAMPLES

The present invention will now be further exemplified with reference to the following example(s). The present invention is in no way limited to the given examples.

### Example 1: Development of the markers

Many genotypes were screened for resistance against BI: 16-36 and a *L. serriola* was found to be resistant against all races.

A segregating F1S1 population was developed to map the resistance the population was derived from the *Bremia* resistant *L. serriola* and a susceptible *L. sativa* was analyzed to identify the genomic location(s) of the resistance. The F1S1 population consisted of 566 seedlings. From those 566 seedlings, 75 were susceptible for B. *lactucae* isolates. Informative genome-wide markers had to be developed to genotype the mapping population. The genotype was determined for 459 F1S1 plants with 232 informative markers. Almost all susceptible plants (69) had an allele from the susceptible parent on chromosome 2 between 6.9 and 11.8 Mbp. The used reference genome was V8 (Reyes-Chin-Wo et al., 2017; https://lgr.genomecenter.ucdavis.edu/). Most resistant plants had at least one allele from the resistant parent in this region.

To make sure the locus on chromosome 2, without any other locus, provided resistance at least against Bl:16-36, the locus was introgressed into a susceptible background. This new line was tested on *Bremia* races Bl:16-36.

It is concluded that the resistance derived from the aforementioned *L. serriola* is a monogenic resistance which localizes on chromosome 2 between 6.9 and 11.8 Mbp in *L. sativa.* The offspring of plants, homozygous for the allele on chromosome 2 between 6.9 and 11.8 Mbp that confers resistance, showed full resistance against seven *Bremia* isolates.

To narrow down the region in which the monogenic R gene is present, two BC3S1 populations of 98 and 85 plants respectively were sown and tested for resistance. The region on chromosome 2 between 6.9 and 11.8 Mbp co-segregated with the disease phenotype and could be narrowed down to a region between 8.4 and 10.5 Mbp.

To further validate the identified genomic region 101 lines were screened for the presence of the resistance locus. Three of the plants contained the resistance and the resistance locus. The others did not contain this specific resistance and lacked the resistance locus, based on marker scores.

Crossing-over was suppressed in the identified locus. This made fine-mapping difficult. Marker development was hampered by the genetic complexity/variability of the identified locus.

Breeding material was screened with eight markers located on chromosome 2 between 8.4 Mbp and 10.5 Mbp.

Subsequently, thousands of plants were tested with markers between 8.4 Mbp and 10.5 Mbp (left and right border of the identified locus). No plants were found in which the molecular results did not correlate with the disease assay results.

### Example 2: Disease test

The tests are performed in a climate chamber with high humidity. Day length is 16 hours and during the light period the temperature is 18 °C and RH about 85 %. During the night (dark period) the temperature is 15 °C and the RH about 100 %. Before inoculation of plants, the spores of the *Bremia* pathogen are propagated on susceptible varieties. The choice of a susceptible variety for a *Bremia* isolate is made from the official differential host set and from a company owned set. Disease testing for *Bremia* resistance was performed using all currently known *Bremia* races or isolates (BI: 16EU to BI: 36EU). Performance of seeds deposied as NCIMB 43292 was compared to currently known *Lactuca* varieties. Table 2 visualizes the reactions of *Bremia* races BI: 16-36 EU.

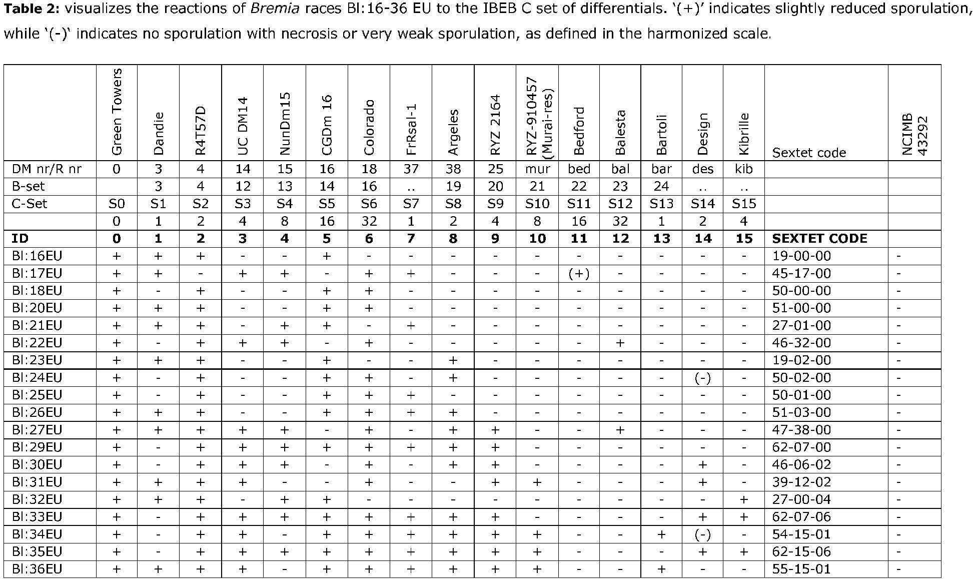

## Claims

1. A nucleic acid fragment comprising a resistance allele from *L. serriola* which confers a broad spectrum resistance to *Bremia lactucae* in *Lactuca sativa,* **characterized in that**, said resistance allele co-segregates with one or more marker sequences chosen from Seq ID No. 1, Seq ID No. 2, Seq ID No. 3, Seq ID No. 4, Seq ID No. 5, Seq ID No. 6, Seq ID No. 7, and/or any *L. serriola* genome specific marker in between Seq ID No. 1 and Seq ID No. 7.

2. Nucleic acid fragment according to claim 1, **characterized in that** said fragment is introduced into a cultivated lettuce (*Lactuca sativa* L.) plant, wherein the resistance allele is located on chromosome 2, preferably between 6.9 and 11.8 Mbp of said chromosome 2.

3. Nucleic acid fragment according to claim 1 or 2, **characterized in that** said resistance allele is as found in the genome of a cultivated lettuce (*Lactuca sativa* L.) plant grown from seeds of which a representative sample was deposited under NCIMB Accession No. NCIMB 43292.

4. A cultivated lettuce (*Lactuca sativa* L.) plant, **characterized in that** said plant comprises said nucleic acid molecule comprising a resistance allele from *L. serriola* which confers a broad spectrum resistance to *Bremia lactucae,* wherein the resistance allele is located on chromosome 2 and co-segregates with one or more sequences chosen from Seq ID No. 1, Seq ID No. 2, Seq ID No. 3, Seq ID No. 4, Seq ID No. 5, Seq ID No. 6, and Seq ID No. 7.

5. Cultivated lettuce (*Lactuca sativa* L.) plant according to claim 4, **characterized in that** said plant confers a broad spectrum resistance at least to *Bremia lactucae* races Bl:16 to BI:36.

6. A cell or tissue from a cultivated lettuce (*Lactuca sativa* L.) plant according to claim 4 or 5, **characterized in that**, said cell and tissue have a broad spectrum resistance to *Bremia lactucae* and said cell and tissue are suitable for culturing.

7. Propagation material derived from a cultivated lettuce plant (*Lactuca sativa* L.) according to claim 4 or 5, **characterized in that** said material has a broad spectrum resistance to *Bremia lactucae,* wherein said material is selected from the group consisting of microspores, pollen, ovaries, ovules, embryos, embryo sacs, egg cells, cuttings, roots, root tips, hypocotyls, cotyledons, stems, leaves, flowers, anthers, seeds, meristematic cells, protoplasts, and cells, and said propagation material is capable of growing into a plant as claimed in claim 4 or 5.

8. Seed capable of growing into a cultivated lettuce plant (*Lactuca sativa* L.) according to claim 4 or 5, **characterized in that**, the progeny plant has a broad spectrum resistance to *Bremia lactucae.*

9. Seed produced on a cultivated lettuce (*Lactuca sativa* L.) plant according to claim 4 or 5, **characterized in that**, the lettuce plant that can be grown from the seed has a broad spectrum resistance to *Bremia lactucae.*

10. Progeny of a cultivated lettuce (*Lactuca sativa* L.) plant according to claim 4 or 5, **characterized in that** the progeny plant has a broad spectrum resistance to *Bremia lactucae.*

11. Use of marker Seq ID No. 1, Seq ID No. 2, Seq ID No. 3, Seq ID No. 4, Seq ID No. 5, Seq ID No. 6, Seq ID No. 7, and/or any *L. serriola* genome specific marker in between Seq ID No. 1 and Seq ID No. 7 to identify or develop broad spectrum *Bremia lactucae* resistant plants, or develop other markers co-segregating with the resistance allele as defined in claims 1-3.

12. A method for production of a cultivated lettuce (*Lactuca sativa* L.) plant **characterized in that**, an introgression from *L. serriola* on chromosome 2 comprising a resistance allele from *L. serriola* which confers a broad spectrum resistance at least to *Bremia lactucae* races BI:16 to BI:36, wherein introgression comprises the sequence of Seq ID No. 1, Seq ID No. 2, Seq ID No. 3, Seq ID No. 4, Seq ID No. 5, Seq ID No. 6, and/or Seq ID No. 7 by using tissue culture.

13. Method according to claim 12 **characterized in that**, said tissue culture comprises cells or tissues according to claim 6 or propagation material according to any of the claims 7-9.

14. A method for production of a cultivated lettuce (*Lactuca sativa* L.) plant characterized that, an introgression from *L. serriola* on chromosome 2 comprising a resistance allele from *L. serriola* which confers a broad spectrum resistance at least to *Bremia lactucae* races BI:16 to BI:36, wherein introgression comprises the sequence of Seq ID No. 1, Seq ID No. 2, Seq ID No. 3, Seq ID No. 4, Seq ID No. 5, Seq ID No. 6, and/or Seq ID No. 7 by using vegetative reproduction.

15. A cultivated plant produced by the method of any of the claims 12-14.
